# EUROPEAN PATENT SPECIFICATION

(11) **EP 3 495 324 B1**
(45) Date of publication and mention of the grant of the patent: **25.08.2021**
(21) Application number: 17836717.3
(22) Date of filing: 13.07.2017
(51) Int. Cl.: A61K 8/25, A61K 8/27, A61K 8/891, A61Q 17/04, A61K 8/02, C01G 9/02, C09C 1/04, C09C 3/12

(54) **ZINC OXIDE-CONTAINING COMPOSITE PARTICLES, ULTRAVIOLET-SHIELDING COMPOSITION, AND COSMETIC**
ZINKOXIDHALTIGE VERBUNDSTOFFPARTIKEL, ZUSAMMENSETZUNG ZUR UV-ABSCHIRMUNG UND KOSMETIKUM
PARTICULES COMPOSITES CONTENANT DE L'OXYDE DE ZINC, COMPOSITION POUR LA PROTECTION CONTRE LES UV ET PRODUIT COSMÉTIQUE

(30) Priority: 04.08.2016 JP 2016153495
(43) Date of publication of application: 12.06.2019
(73) Proprietor: Nippon Sheet Glass Company, Limited, Tokyo 108-6321 (JP)
(72) Inventor: DOSHITA Kazuhiro, Tokyo 108-6321 (JP); SHIMOKAWA Kosei, Tokyo 108-6321 (JP)
(74) Representative: Cabinet Beau de Loménie
(86) International application number: PCT/JP2017/025623
(87) International publication number: WO 2018/025610

(56) References cited:
- WO-A1-2008/129901
- WO-A1-2016/189828
- JP-A- H11 193 354
- JP-A- 2000 319 128
- JP-A- 2007 023 127
- KR-A- 20140 088 391
- KOICHIRO MAKITA et al.: "Development of functional materials for cosmetics using sol- gel method", Shikizai Kenkyu Happyokai Koen Yoshishu, vol. 1999, 12 October 1999 (1999-10-12), pages 78-79, XP009518380,

## Description

### TECHNICAL FIELD

The present invention relates to zinc oxide-containing composite particles, an ultraviolet-shielding composition, and a cosmetic.

### BACKGROUND ART

Zinc oxide (ZnO) has conventionally been incorporated in a cosmetic, for example, as a shielding agent against UV-A (ultraviolet light with wavelengths of 320 nm to 400 nm). Zinc oxide, being an oxide of zinc which is an amphoteric element, is easily soluble in both acidic solutions and basic solutions and is also soluble, albeit slightly, even in water which is nearly neutral. Dissolution of zinc ions from zinc oxide may deteriorate some of the properties of a zinc oxide-containing product such as a cosmetic. Various techniques have therefore been proposed to reduce the ion dissolution from zinc oxide.

For example, Patent Literature 1 describes composite particles for cosmetics, the particles containing a specific polyolefin resin and zinc oxide.

Patent Literature 2 describes a surface-coated zinc oxide material including zinc oxide particles, a titania-containing coating on the surface of the zinc oxide particles, and a silica-containing coating on the titania-containing coating.

Patent Literature 3 describes a method for producing a surface-coated zinc oxide material, the method including the steps of: obtaining zinc oxide particles by reacting a zinc compound with a neutralizer in an aqueous medium; and coating the surface of the obtained zinc oxide particles with an inorganic compound such as silica in the aqueous medium without heating the zinc oxide particles in a gaseous phase.

Patent Literature 4 describes a zinc oxide powder coated with water-repellent particulate silica, the powder being obtained by dispersing a zinc oxide powder in a solution of a specific acrylic/silicone resin, then adding a volatile alkaline substance and a specific alkoxysilane to the resulting dispersion, and hydrolyzing the alkoxysilane.

Patent Literatures 5 and 6 each describe coated zinc oxide particles having a coating layer made of a precipitable material obtained by reaction of a carboxyvinyl polymer with a divalent or trivalent metal ion.

Patent Literature 7 describes a zinc oxide-based composite powder including a zinc oxide powder and a dipeptide that binds to zinc ions to prevent the zinc ions from dissolving into water.

Patent Literature 8 describes ultraviolet-shielding composite particles including: a core containing zinc oxide and a first resin; and a coating film provided on the surface of the core, the coating film being made of a second resin having a composition identical to or different from the composition of the first resin.

Patent Literature 9 describes a silicon oxide-coated zinc oxide material including zinc oxide particles with an average particle diameter of 1 nm or more and 50 nm or less and a specific silicon oxide coating provided on the surface of the zinc oxide particles.

Patent Literature 10, although making no mention of inhibition of dissolution of zinc ions from zinc oxide, describes silica-coated fine zinc oxide particles including fine zinc oxide particles with an average particle diameter of 5 to 100 nm as a base material, the base material being coated with silica, which amounts to 15 to 40 mass% relative to the base material.

### CITATION LIST

### Patent Literature

Patent Literature 1: JP 2006-219437 A
Patent Literature 2: JP 2008-94917 A
Patent Literature 3: JP 2008-273760 A
Patent Literature 4: JP 2009-23981 A
Patent Literature 5: JP 2011-102291 A
Patent Literature 6: JP 2012-207039 A
Patent Literature 7: JP 2014-43376 A
Patent Literature 8: JP 2014-84448 A
Patent Literature 9: JP 2015-6976 A
Patent Literature 10: JP 2007-016111 A KR20140088391 A discloses in ex. 5 a ZnO / silica / polysilsesquioxane particle for sunscreen.

### SUMMARY OF INVENTION

### Technical Problem

The techniques described in Patent Literatures 2 and 3 take into account the acid resistance of surface-coated zinc oxide materials. However, the acid resistance of composite particles containing zinc oxide has not been fully considered and there is still room for designing zinc oxide-containing composite particles having acid resistance from a new point of view. It is therefore an object of the present invention to provide new zinc oxide-containing composite particles having high acid resistance and capable of maintaining acid resistance even upon contact with water.

### Solution to Problem

The present invention provides zinc oxide-containing composite particles each including:
a dense matrix formed of silica and a polysilsesquioxane; and
zinc oxide particles dispersed within the matrix, wherein
the content of the polysilsesquioxane in the matrix is 30 mass% to 70 mass%.

The present invention also provides an ultraviolet-shielding composition including the zinc oxide-containing composite particles.

The present invention also provides a cosmetic including the zinc oxide-containing composite particles.

### Advantageous Effects of Invention

By virtue of having zinc oxide particles dispersed within a dense matrix formed of silica and a polysilsesquioxane, the zinc oxide-containing composite particles have high acid resistance and are capable of maintaining acid resistance even upon contact with water. An ultraviolet-shielding composition or a cosmetic including zinc oxide-containing composite particles having high acid resistance can also be provided.

### BRIEF DESCRIPTION OF DRAWING

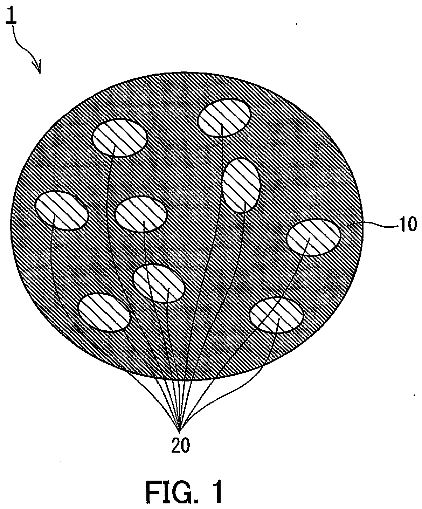

FIG. 1 is a cross-sectional view schematically illustrating the structure of a zinc oxide-containing composite particle according to an embodiment of the present invention.

### DESCRIPTION OF EMBODIMENTS

Hereinafter, an embodiment of the present invention will be described with reference to the drawings. The following description concerns examples of the present invention, and the present invention is not limited to these examples.

As shown in FIG. 1, a zinc oxide-containing composite particle 1 includes a dense matrix 10 and zinc oxide particles 20. The dense matrix 10 is formed of silica and a polysilsesquioxane. The zinc oxide particles 20 are dispersed within the matrix 10. The content of the polysilsesquioxane in the matrix 10 is 30 mass% to 70 mass%.

The matrix 10 is dense and exhibits a water-repellent effect attributed to the polysilsesquioxane. Thus, even when the zinc oxide-containing composite particles 1 contact water, the zinc oxide particles 20 dispersed within the matrix 10 are unlikely to contact water. This reduces the dissolution of zinc from the zinc oxide particles 20 upon contact of the zinc oxide-containing composite particles 1 with water, enabling the zinc oxide-containing composite particles 1 to have a high long-term stability when incorporated in an aqueous cosmetic such as that containing a carboxyvinyl polymer. Additionally, the silica contained in the matrix 10 offers a hydrophilic effect. Thus, the zinc oxide-containing composite particles 1 can be well dispersed in water thanks to the hydrophilic effect offered by the silica while exhibiting acid resistance due to the water-repellent effect offered by the polysilsesquioxane. In order to obtain both high long-term stability (acid resistance) of the zinc oxide-containing composite particles 1 and high water dispersibility of the zinc oxide-containing composite particles 1, the content of the polysilsesquioxane in the matrix 10 is, for example, within the range mentioned above and is desirably 40 mass% to 70 mass%, more desirably 50 mass% to 60 mass%.

The content of the polysilsesquioxane in the matrix 10 can be determined, for example, by a spectroscopic technique such as Fourier transformation infrared spectroscopy (FT-IR) or Si-NMR, a chemical technique based on elemental analysis, or a thermoanalytical technique such as thermogravimetry (TG).

The silica included in the matrix 10 is formed, for example, by hydrolysis and dehydration condensation of a tetrafunctional alkoxysilane. The polysilsesquioxane included in the matrix 10 is formed, for example, by hydrolysis and dehydration condensation of a trifunctional alkoxysilane. Thus, the matrix 10 is formed, for example, using a sol-gel process. This is why the matrix 10 is likely to have a dense structure.

The polysilsesquioxane included in the matrix 10 is, for example, a polysilsesquioxane having an alkyl group bonded to a silicon atom, the alkyl group having 16 or less carbon atoms. In this case, the matrix 10 is more likely to be dense and capable of exhibiting an appropriate water-repellent effect. This ensures that the zinc oxide-containing composite particles 1 have high acid resistance and exhibit long-term stability even upon contact with water. Additionally, good dispersion of the zinc oxide-containing composite particles 1 in water can be ensured. The alkyl group may be linear or branched.

The polysilsesquioxane included in the matrix 10 desirably includes at least one polysilsesquioxane selected from the group consisting of polymethylsilsesquioxane, polyethylsilsesquioxane, and polypropylsilsesquioxane. The polymethylsilsesquioxane is a polysiloxane having a basic structural unit having one methyl group bonded to one silicon atom. The polyethylsilsesquioxane is a polysiloxane having a basic structural unit having one ethyl group bonded to one silicon atom. The polypropylsilsesquioxane is a polysiloxane having a basic structural unit having one 1-propyl group or one 2-propyl group bonded to one silicon atom. In this case, it is ensured that the zinc oxide-containing composite particles 1 have high acid resistance and exhibit long-term stability even upon contact with water. Additionally, good dispersion of the zinc oxide-containing composite particles 1 in water can be ensured.

By virtue of the matrix 10 having a dense structure, the zinc oxide-containing composite particles 1 have a small specific surface area. For example, the specific surface area of the zinc oxide-containing composite particles 1, as measured by a nitrogen adsorption method, is 5 m²/g or less. In this case, it is ensured that the zinc oxide-containing composite particles 1 have high acid resistance and exhibit long-term stability even upon contact with water. Additionally, good dispersion of the zinc oxide-containing composite particles 1 in water can be ensured.

The zinc oxide-containing composite particle 1 are not particularly limited in shape and are, for example, spherical. The term "spherical" as used herein means that the zinc oxide-containing composite particle 1 has the shape of a granule in which the ratio of its largest diameter to its smallest diameter is in the range of 1 to 1.5. When the zinc oxide-containing composite particles 1 are spherical, the zinc oxide-containing composite particles 1 can be incorporated in a product in such a manner as to provide the product with desired properties depending on the purpose of the product.

The average particle diameter of the zinc oxide-containing composite particles 1 is, for example, 3 µm to 15 µm. The average particle diameter of the zinc oxide-containing composite particles 1, as defined herein, refers to a particle diameter at cumulative volume of 50% (D50) in a particle size distribution measured with a laser diffraction particle size analyzer.

The average particle diameter (average secondary particle diameter) of the zinc oxide particles 20 is not particularly limited as long as the average particle diameter is smaller than the smallest diameter of the matrix 10. For example, the average particle diameter of the zinc oxide particles 20 is 10 nm to 1000 nm. In this case, the zinc oxide particles 20 are easily dispersed within the matrix 10 in an appropriate manner. Thus, even upon contact of the zinc oxide-containing composite particles 1 with water, dissolution of zinc ions from the zinc oxide particles 20 can be prevented. The average particle diameter of the zinc oxide particles 20, as defined herein, refers to a particle diameter at cumulative volume of 50% (D50) in a particle size distribution measured with a laser diffraction particle size analyzer.

The content of the zinc oxide particles 20 in the zinc oxide-containing composite particle 1 is, for example, 10 mass% to 50 mass%. In this case, the zinc oxide-containing composite particles 1 are more likely to exhibit desired properties.

The zinc oxide particles 20 may, if necessary, be zinc oxide particles having a surface coated with a material such as silica. In this case, the silica derived from the zinc oxide particles 20 should be distinguished from the silica of the matrix 10.

Zinc oxide can act as a UV-A shielding agent; thus, the use of the zinc oxide-containing composite particles 1 can provide an ultraviolet-shielding composition including the zinc oxide-containing composite particles 1.

The use of the zinc oxide-containing composite particles 1 can also provide a cosmetic including the zinc oxide-containing composite particles 1. Thanks to the inclusion of the zinc oxide-containing composite particles 1, this cosmetic has the property of shielding against UV-A. The cosmetic is, for example, an O/W-type aqueous cosmetic containing a carboxyvinyl polymer. In this case, the presence of the carboxyvinyl polymer allows control of the viscosity of the cosmetic within a desired range.

For example, if zinc oxide is incorporated into an O/W-type aqueous cosmetic having an aqueous phase containing a carboxyvinyl polymer as a viscosity modifier, dissolved zinc ions may interact with the carboxyvinyl polymer to decrease the viscosity of the system. For this reason, the incorporation of zinc oxide into aqueous cosmetics is not feasible in many cases. However, the zinc oxide-containing composite particles 1 have high acid resistance. Thus, when the zinc oxide-containing composite particles 1 are incorporated into an O/W-type aqueous cosmetic containing a carboxyvinyl polymer, dissolution of zinc ions from the zinc oxide particles 20 and hence interaction of zinc ions with the carboxyvinyl polymer are prevented. The use of the zinc oxide-containing composite particles 1 in a cosmetic therefore allows both a carboxyvinyl polymer as a viscosity modifier and zinc oxide to be present in the cosmetic in a preferred manner without dissolution of zinc ions. The zinc oxide-containing composite particles 1 can, as described above, be incorporated into an O/W-type aqueous cosmetic containing a carboxyvinyl polymer, and this incorporation enables the cosmetic to have a desired viscosity and also to maintain the UV-A shielding property over a long period of time.

Any of the following substances may be deposited or adsorbed on the surface of the zinc oxide-containing composite particles according to the present invention: amphiphilic materials such as a surfactant; and hydrophilic polymers such as a silane compound having a polyether group, polyvinyl alcohol, polyethylene glycol, and polyvinylpyrrolidone. In this case, the dispersibility of the zinc oxide-containing composite particles in water is adjusted.

An example of the method for producing the zinc oxide-containing composite particles according to the present invention will now be described. First, a tetrafunctional alkoxysilane such as tetraethyl orthosilicate (tetraethoxysilane: TEOS), a trifunctional alkoxysilane such as methyltrimethoxysilane, a hydrolysis catalyst such as acetic acid, and pure water are mixed. This mixture is stirred at a given temperature for a given period of time to prepare a sol for forming the matrix. The sol for forming the matrix contains solids including silica resulting from hydrolysis and dehydration condensation of the tetrafunctional alkoxysilane and a polysilsesquioxane resulting from hydrolysis and dehydration condensation of the trifunctional alkoxysilane.

Next, zinc oxide particles are dispersed in the sol for forming the matrix. For this purpose, a powder of the zinc oxide particles may be added directly to the sol for forming the matrix. Desirably, a dispersion of the zinc oxide particles is prepared beforehand using a wet bead mill or like means and is mixed and stirred with the sol for forming the matrix to give a sol for forming the composite particles. The surface of the zinc oxide particles to be added to the sol for forming the matrix may be surface-treated with silica depending on the situation. This allows the zinc oxide particles to be easily dispersed in the matrix. Next, solids in the sol for forming the composite particles are granulated. This process of granulation is not particularly limited and may be carried out, for example, by spray drying. In this case, spherical zinc oxide-containing composite particles can be easily obtained. The temperature of the drying oven used in the spray drying is, for example, 150 to 220°C. The conditions of the spray drying are adjusted so that the resulting zinc oxide-containing composite particles will have an average particle diameter of 3 to 15 µm. The zinc oxide-containing composite particles according to the present invention can be produced in this manner, for example.

### Examples

The present invention will be described in more detail with examples. The present invention is not limited to the examples presented below.

### <Example 1>

To 70 parts by mass of pure water were added 30 parts by mass of zinc oxide particles (manufactured by Tayca Corporation, product name: MZ-500HP, zinc oxide content: 80 mass%, silica content: 20 mass%), and the mixture was stirred and circulated together with 4.3 kg of 0.65-mm-diameter zirconia beads using a horizontal, continuous-type wet stirred-media mill (manufactured by Shinmaru Enterprises Corporation, product name: "DYNO-MILL KDL-PILOT A") for 3 hours (stirring speed: peripheral speed = 10 m/sec, flow speed = 5 L/min), and thus a dispersion of zinc oxide particles (diameter of dispersed zinc oxide particles: about 0.2 pm) was obtained. MZ-500HP consists of zinc oxide particles having a silica-coated surface.

117.05 parts by mass of ion-exchanged water, 5 parts by mass of 1 mass% acetic acid, 38.03 parts by mass of methyltrimethoxysilane (manufactured by Tama Chemicals Co., Ltd.), and 64.92 parts by mass of tetraethyl orthosilicate (ethyl orthosilicate manufactured by Tama Chemicals Co., Ltd.) were mixed, and the mixture was stirred at 25°C for about 20 hours to obtain a transparent sol A. The sol A contained polymethylsilsesquioxane as a solid derived from methyltrimethoxysilane and silica as a solid derived from tetraethyl orthosilicate. The ratio between the mass Ma of polymethylsilsesquioxane and the mass Mb of silica in the sol A was 5:5 (Ma:Mb).

225 parts by mass of the sol A and 75 parts by mass of the dispersion of zinc oxide particles were mixed to obtain a sol B. A spray dryer (manufactured by Fujisaki Electric Co., Ltd., product name: MDL-050L) was used to spray-dry the sol B in a 200°C atmosphere to produce zinc oxide-containing composite particles according to Example 1. Zinc oxide particles surface-treated with silica (zinc oxide: 80 mass%, silica: 20 mass%) were contained within the zinc oxide-containing composite particles according to Example 1. The content of the zinc oxide particles in the zinc oxide-containing composite particles according to Example 1 was 37.5 mass%.

Observation of the zinc oxide-containing composite particles according to Example 1 with an optical microscope demonstrated that the zinc oxide-containing composite particles according to Example 1 were spherical particles. Measurement of the particle size distribution of the zinc oxide-containing composite particles according to Example 1 by means of a laser diffraction particle size analyzer revealed that the diameter at cumulative volume of 50% (D50) of the zinc oxide-containing composite particles according to Example 1 was about 10 µm. Measurement of the pore distribution of the zinc oxide-containing composite particles according to Example 1 by means of a nitrogen adsorption method suggested that the zinc oxide-containing composite particles according to Example 1 were almost free of pores and had a dense structure. The specific surface area of the zinc oxide-containing composite particles according to Example 1, as measured by the nitrogen adsorption method, was 1.1 m²/g.

0.68 parts by mass of the zinc oxide-containing composite particles according to Example 1 and 11.32 parts by mass of ion-exchanged water were mixed, and the mixture was stirred for 3 hours to obtain a composite particle dispersion according to Example 1. 1 part by mass of the composite particle dispersion according to Example 1 was added to 10 parts by mass of a 0.1 mass% aqueous citric acid solution with a pH of 2.5, and the mixture was stirred to disperse the zinc oxide-containing composite particles according to Example 1 in the aqueous citric acid solution. The zinc oxide-containing composite particles according to Example 1 were uniformly dispersed in the aqueous citric acid solution. The pH of the aqueous solution was about 3.4 at 1 hour after the zinc oxide-containing composite particles according to Example 1 were dispersed. Thereafter, the pH of the aqueous solution remained almost unchanged. This leads to the conclusion that little amount of zinc ions were dissolved from the zinc oxide contained within the zinc oxide-containing composite particles according to Example 1.

### <Example 2>

122.42 parts by mass of ion-exchanged water, 5 parts by mass of 1 mass% acetic acid, 45.64 parts by mass of methyltrimethoxysilane (manufactured by Tama Chemicals Co., Ltd.), and 51.94 parts by mass of tetraethyl orthosilicate (ethyl orthosilicate manufactured by Tama Chemicals Co., Ltd.) were mixed, and the mixture was stirred at 25°C for about 20 hours to obtain a transparent sol C. In the sol C, the ratio between the mass Ma of polymethylsilsesquioxane contained as a solid derived from methyltrimethoxysilane and the mass Mb of silica contained as a solid derived from tetraethyl orthosilicate was 6:4 (Ma:Mb).

225 parts by mass of the sol C and 75 parts by mass of a dispersion of zinc oxide particles as used in Example 1 were mixed to obtain a sol D. A spray dryer (manufactured by Fujisaki Electric Co., Ltd., product name: MDL-050L) was used to spray-dry the sol D in a 200°C atmosphere to produce zinc oxide-containing composite particles according to Example 2. Zinc oxide particles surface-treated with silica (zinc oxide: 80 mass%, silica: 20 mass%) were contained within the zinc oxide-containing composite particles according to Example 2. The content of the zinc oxide particles in the zinc oxide-containing composite particles according to Example 2 was 37.5 mass%.

Observation of the zinc oxide-containing composite particles according to Example 2 with an optical microscope demonstrated that the zinc oxide-containing composite particles according to Example 2 were spherical particles. Measurement of the particle size distribution of the zinc oxide-containing composite particles according to Example 2 by means of a laser diffraction particle size analyzer revealed that the diameter at cumulative volume of 50% (D50) of the zinc oxide-containing composite particles according to Example 2 was about 10 µm. Measurement of the pore distribution of the zinc oxide-containing composite particles according to Example 2 by means of a nitrogen adsorption method suggested that the zinc oxide-containing composite particles according to Example 2 were almost free of pores and had a dense structure. The specific surface area of the zinc oxide-containing composite particles according to Example 2, as measured by the nitrogen adsorption method, was 0.9 m²/g.

0.68 parts by mass of the zinc oxide-containing composite particles according to Example 2 and 11.32 parts by mass of ion-exchanged water were mixed, and the mixture was stirred for 3 hours to obtain a composite particle dispersion according to Example 2. 1 part by mass of the composite particle dispersion according to Example 2 was added to 10 parts by mass of a 0.1 mass% aqueous citric acid solution with a pH of 2.5, and the mixture was stirred to disperse the zinc oxide-containing composite particles according to Example 2 in the aqueous citric acid solution. The zinc oxide-containing composite particles according to Example 2 were uniformly dispersed in the aqueous citric acid solution. The pH of the aqueous solution was about 3.3 at 1 hour after the zinc oxide-containing composite particles according to Example 2 were dispersed. Thereafter, the pH of the aqueous solution remained almost unchanged. This leads to the conclusion that little amount of zinc ions were dissolved from the zinc oxide contained within the zinc oxide-containing composite particles according to Example 2.

### <Example 3>

111.67 parts by mass of ion-exchanged water, 5 parts by mass of 1 mass% acetic acid, 30.42 parts by mass of methyltrimethoxysilane (manufactured by Tama Chemicals Co., Ltd.), and 77.91 parts by mass of tetraethyl orthosilicate (ethyl orthosilicate manufactured by Tama Chemicals Co., Ltd.) were mixed, and the mixture was stirred at 25°C for about 20 hours to obtain a transparent sol E. In the sol E, the ratio between the mass Ma of polymethylsilsesquioxane contained as a solid derived from methyltrimethoxysilane and the mass Ma of silica contained as a solid derived from tetraethyl orthosilicate was 4:6 (Ma:Mb).

225 parts by mass of the sol E and 75 parts by mass of a dispersion of zinc oxide particles as used in Example 1 were mixed to obtain a sol F. A spray dryer (manufactured by Fujisaki Electric Co., Ltd., product name: MDL-050L) was used to spray-dry the sol F in a 200°C atmosphere to produce zinc oxide-containing composite particles according to Example 3. Zinc oxide particles surface-treated with silica (zinc oxide: 80 mass%, silica: 20 mass%) were contained within the zinc oxide-containing composite particles according to Example 3. The content of the zinc oxide particles in the zinc oxide-containing composite particles according to Example 3 was 37.5 mass%.

Observation of the zinc oxide-containing composite particles according to Example 3 with an optical microscope demonstrated that the zinc oxide-containing composite particles according to Example 3 were spherical particles. Measurement of the particle size distribution of the zinc oxide-containing composite particles according to Example 3 by means of a laser diffraction particle size analyzer revealed that the diameter at cumulative volume of 50% (D50) of the zinc oxide-containing composite particles according to Example 3 was about 10 µm. Measurement of the pore distribution of the zinc oxide-containing composite particles according to Example 3 by means of a nitrogen adsorption method suggested that the zinc oxide-containing composite particles according to Example 3 were almost free of pores and had a dense structure. The specific surface area of the zinc oxide-containing composite particles according to Example 3, as measured by the nitrogen adsorption method, was 2.0 m²/g.

0.68 parts by mass of the zinc oxide-containing composite particles according to Example 3 and 11.32 parts by mass of ion-exchanged water were mixed, and the mixture was stirred for 3 hours to obtain a composite particle dispersion according to Example 3. 1 part by mass of the composite particle dispersion according to Example 3 was added to 10 parts by mass of a 0.1 mass% aqueous citric acid solution with a pH of 2.5, and the mixture was stirred to disperse the zinc oxide-containing composite particles according to Example 3 in the aqueous citric acid solution. The zinc oxide-containing composite particles according to Example 3 were uniformly dispersed in the aqueous citric acid solution. The pH of the aqueous solution was about 3.8 at 1 hour after the zinc oxide-containing composite particles according to Example 3 were dispersed. Thereafter, the pH of the aqueous solution remained almost unchanged. This suggested that the zinc oxide-containing composite particles according to Example 3 maintained sufficient acid resistance although there was a possibility of slight dissolution of zinc ions from the zinc oxide contained within the zinc oxide-containing composite particles.

### <Example 4>

127.80 parts by mass of ion-exchanged water, 5 parts by mass of 1 mass% acetic acid, 53.24 parts by mass of methyltrimethoxysilane (manufactured by Tama Chemicals Co., Ltd.), and 38.95 parts by mass of tetraethyl orthosilicate (ethyl orthosilicate manufactured by Tama Chemicals Co., Ltd.) were mixed, and the mixture was stirred at 25°C for about 20 hours to obtain a transparent sol G. In the sol G, the ratio between the mass Ma of polymethylsilsesquioxane contained as a solid derived from methyltrimethoxysilane and the mass Mb of silica contained as a solid derived from tetraethyl orthosilicate was 7:3 (Ma:Mb).

225 parts by mass of the sol G and 75 parts by mass of a dispersion of zinc oxide particles as used in Example 1 were mixed to obtain a sol H. A spray dryer (manufactured by Fujisaki Electric Co., Ltd., product name: MDL-050L) was used to spray-dry the sol H in a 220°C atmosphere to produce zinc oxide-containing composite particles according to Example 4. Zinc oxide particles surface-treated with silica (zinc oxide: 80 mass%, silica: 20 mass%) were contained within the zinc oxide-containing composite particles according to Example 4. The content of the zinc oxide particles in the zinc oxide-containing composite particles according to Example 4 was 37.5 mass%.

Observation of the zinc oxide-containing composite particles according to Example 4 with an optical microscope demonstrated that the zinc oxide-containing composite particles according to Example 4 were spherical particles. Measurement of the particle size distribution of the zinc oxide-containing composite particles according to Example 4 by means of a laser diffraction particle size analyzer revealed that the diameter at cumulative volume of 50% (D50) of the zinc oxide-containing composite particles according to Example 4 was about 10 µm. Measurement of the pore distribution of the zinc oxide-containing composite particles according to Example 4 by means of a nitrogen adsorption method suggested that the zinc oxide-containing composite particles according to Example 4 were almost free of pores and had a dense structure. The specific surface area of the zinc oxide-containing composite particles according to Example 4, as measured by the nitrogen adsorption method, was 0.7 m²/g.

0.68 parts by mass of the zinc oxide-containing composite particles according to Example 4 and 11.32 parts by mass of ion-exchanged water were mixed, and the mixture was stirred for 3 hours to obtain a composite particle dispersion according to Example 4. 1 part by mass of the composite particle dispersion according to Example 4 was added to 10 parts by mass of a 0.1 mass% aqueous citric acid solution with a pH of 2.5, and the mixture was stirred to disperse the zinc oxide-containing composite particles according to Example 4 in the aqueous citric acid solution. The zinc oxide-containing composite particles according to Example 4 were almost uniformly dispersed in the aqueous citric acid solution. The pH of the aqueous solution was about 3.2 at 1 hour after the zinc oxide-containing composite particles according to Example 4 were dispersed. Thereafter, the pH of the aqueous solution remained almost unchanged. This leads to the conclusion that little amount of zinc ions were dissolved from the zinc oxide contained within the zinc oxide-containing composite particles according to Example 2.

### <Comparative Example 1>

0.26 parts by mass of silica-coated zinc oxide particles (manufactured by Tayca Corporation, product name: MZ-500HP, zinc oxide content: 80 mass%, silica content: 20 mass%) and 11.74 parts by mass of ion-exchanged water were mixed, and the mixture was stirred for 3 hours to obtain a particle dispersion according to Comparative Example 1. 1 part by mass of the particle dispersion according to Comparative Example 1 was added to 10 parts by mass of a 0.1 mass% aqueous citric acid solution with a pH of 2.5, and the mixture was stirred to disperse the silica-coated zinc oxide particles in the aqueous citric acid solution. The pH of the aqueous solution immediately increased to 6 or higher, which confirmed dissolution of zinc ions.

### <Comparative Example 2>

0.26 parts by mass of octyltriethoxysilane-treated zinc oxide (manufactured by Tayca Corporation, product name: MZX-508OTS) and 11.74 parts by mass of ion-exchanged water were mixed, and the mixture was stirred for 3 hours. The octyltriethoxysilane-treated zinc oxide kept floating on the water surface and was not dispersed in the water even by vigorous stirring.

### <Comparative Example 3>

133.17 parts by mass of ion-exchanged water, 5 parts by mass of 1 mass% acetic acid, 60.87 parts by mass of methyltrimethoxysilane (manufactured by Tama Chemicals Co., Ltd.), and 25.96 parts by mass of tetraethyl orthosilicate (ethyl orthosilicate manufactured by Tama Chemicals Co., Ltd.) were mixed, and the mixture was stirred at 25°C for about 20 hours to obtain a transparent sol W. In the sol W, the ratio between the mass Ma of polymethylsilsesquioxane contained as a solid derived from methyltrimethoxysilane and the mass Mb of silica contained as a solid derived from tetraethyl orthosilicate was 8:2 (Ma:Mb).

225 parts by mass of the sol W and 75 parts by mass of a dispersion of zinc oxide particles as used in Example 1 were mixed to obtain a sol X. A spray dryer (manufactured by Fujisaki Electric Co., Ltd., product name: MDL-050L) was used to spray the sol X into a 220°C atmosphere. The droplets of the sol got attached to the wall surface of the dryer, and the sol failed to become granulated.

### <Comparative Example 4>

100.91 parts by mass of ion-exchanged water, 5 parts by mass of 1 mass% acetic acid, 15.21 parts by mass of methyltrimethoxysilane (manufactured by Tama Chemicals Co., Ltd.), and 103.88 parts by mass of tetraethyl orthosilicate (ethyl orthosilicate manufactured by Tama Chemicals Co., Ltd.) were mixed, and the mixture was stirred at 25°C for about 20 hours to obtain a transparent sol Y. In the sol Y, the ratio between the mass of polymethylsilsesquioxane contained as a solid derived from methyltrimethoxysilane and the mass of silica contained as a solid derived from tetraethyl orthosilicate was 2:8.

225 parts by mass of the sol Y and 75 parts by mass of a dispersion of zinc oxide particles as used in Example 1 were mixed to obtain a sol Z. This sol Z turned into a gel in a short time. Before the gelation of the sol Z, a spray dryer (manufactured by Fujisaki Electric Co., Ltd., product name: MDL-050L) was used to spray-dry the sol Z in a 200°C atmosphere to produce zinc oxide-containing composite particles according to Comparative Example 4. Zinc oxide particles surface-treated with silica (zinc oxide: 80 mass%, silica: 20 mass%) were contained within the zinc oxide-containing composite particles according to Comparative Example 4. The content of the zinc oxide particles in the zinc oxide-containing composite particles according to Comparative Example 4 was 37.5 mass%.

Observation of the zinc oxide-containing composite particles according to Comparative Example 4 with an optical microscope demonstrated that the zinc oxide-containing composite particles according to Comparative Example 4 were spherical particles. Measurement of the particle size distribution of the zinc oxide-containing composite particles according to Comparative Example 4 by means of a laser diffraction particle size analyzer revealed that the diameter at cumulative volume of 50% (D50) of the zinc oxide-containing composite particles according to Comparative Example 4 was about 10 µm.

0.68 parts by mass of the zinc oxide-containing composite particles according to Comparative Example 4 and 11.32 parts by mass of ion-exchanged water were mixed, and the mixture was stirred for 3 hours to obtain a composite particle dispersion according to Comparative Example 4. 1 part by mass of the composite particle dispersion according to Comparative Example 4 was added to 10 parts by mass of a 0.1 mass% aqueous citric acid solution with a pH of 2.5, and the mixture was stirred to disperse the zinc oxide-containing composite particles according to Comparative Example 4 in the aqueous citric acid solution. The zinc oxide-containing composite particles according to Comparative Example 4 were uniformly dispersed in the aqueous citric acid solution. The pH of the aqueous solution increased to 6 or higher immediately after the zinc oxide-containing composite particles according to Comparative Example 4 were dispersed, and this confirmed dissolution of zinc ions.

## Claims

1. Zinc oxide-containing composite particles (1) each comprising:
a dense matrix (10) formed of silica and a polysilsesquioxane; and
zinc oxide particles (20) dispersed within the matrix, wherein
the content of the polysilsesquioxane in the matrix is 30 mass% to 70 mass%.

2. The zinc oxide-containing composite particles (1) according to claim 1, wherein
the silica is formed by hydrolysis and dehydration condensation of a tetrafunctional alkoxysilane, and
the polysilsesquioxane is formed by hydrolysis and dehydration condensation of a trifunctional alkoxysilane.

3. The zinc oxide-containing composite particles (1) according to claim 1 or 2, wherein the polysilsesquioxane is a polysilsesquioxane having an alkyl group bonded to a silicon atom, the alkyl group having 16 or less carbon atoms.

4. The zinc oxide-containing composite particles (1) according to claim 1, wherein the polysilsesquioxane comprises at least one polysilsesquioxane selected from the group consisting of polymethylsilsesquioxane, polyethylsilsesquioxane, and polypropylsilsesquioxane.

5. The zinc oxide-containing composite particles (1) according to any one of claims 1 to 4, wherein a specific surface area as measured by a nitrogen adsorption method is 5 m²/g or less.

6. The zinc oxide-containing composite particles (1) according to any one of claims 1 to 5, wherein the zinc oxide-containing composite particles (1) are spherical.

7. The zinc oxide-containing composite particles (1) according to any one of claims 1 to 6, wherein the zinc oxide particles (20) have an average particle diameter of 10 nm to 1000 nm.

8. An ultraviolet-shielding composition comprising the zinc oxide-containing composite particles (1) according to any one of claims 1 to 7.

9. A cosmetic comprising the zinc oxide-containing composite particles (1) according to any one of claims 1 to 7.

10. Ultraviolet-shielding composition according to claim 8, or cosmetic according to claim 9, further comprising a carboxyvinyl polymer.

11. Ultraviolet-shielding composition, or cosmetic, according to claim 10, in the form of an O/W-type aqueous cosmetic.

12. Zinc oxide-containing composite particles according to any of claims 1 to 7 for use in a therapeutic method of ultraviolet shielding.

## Patentansprüche

1. Zinkoxidhaltige Verbundpartikel (1), jeweils umfassend:
eine dichte Matrix (10), gebildet aus Siliciumdioxid und einem Polysilsesquioxan, und
Zinkoxidpartikel (20), die innerhalb der Matrix gelöst sind, wobei der Gehalt des Polysilsesquioxans in der Matrix 30 Masse-% bis 70 Masse-% beträgt.

2. Zinkoxidhaltige Verbundpartikel (1) nach Anspruch 1, wobei
das Siliciumdioxid durch Hydrolyse und Dehydratisierungskondensation eines tetrafunktionalen Alkoxysilans gebildet ist und
das Polysilsesquioxan durch Hydrolyse und Dehydratisierungskondensation eines trifunktionalen Alkoxysilans gebildet ist.

3. Zinkoxidhaltige Verbundpartikel (1) nach Anspruch 1 oder 2, wobei das Polysilsesquioxan ein Polysilsesquioxan mit einer Alkylgruppe ist, die an ein Siliziumatom gebunden ist, wobei die Alkylgruppe 16 oder weniger Kohlenstoffatome aufweist.

4. Zinkoxidhaltige Verbundpartikel (1) nach Anspruch 1, wobei das Polysilsesquioxan zumindest ein Polysilsesquioxan, ausgewählt aus der Gruppe bestehend aus Polymethylsilsesquioxan, Polyethylsilsesquioxan und Polypropylsilsesquioxan, umfasst.

5. Zinkoxidhaltige Verbundpartikel (1) nach einem der Ansprüche 1 bis 4, wobei eine spezifische Oberfläche, wie durch ein Stickstoffadsorptionsverfahren gemessen, 5 m²/g oder weniger beträgt.

6. Zinkoxidhaltige Verbundpartikel (1) nach einem der Ansprüche 1 bis 5, wobei die zinkoxidhaltigen Verbundpartikel (1) kugelförmig sind.

7. Zinkoxidhaltige Verbundpartikel (1) nach einem der Ansprüche 1 bis 6, wobei die Zinkoxidpartikel (20) einen mittleren Partikeldurchmesser von 10 nm bis 1000 nm aufweisen.

8. Ultraviolett-abschirmende Zusammensetzung, umfassend die zinkoxidhaltigen Verbundpartikel (1) nach einem der Ansprüche 1 bis 7.

9. Kosmetikum, umfassend die zinkoxidhaltigen Verbundpartikel (1) nach einem der Ansprüche 1 bis 7.

10. Ultraviolett-abschirmende Zusammensetzung nach Anspruch 8 oder Kosmetikum nach Anspruch 9, ferner umfassend ein Carboxyvinylpolymer.

11. Ultraviolett-abschirmende Zusammensetzung oder Kosmetikum nach Anspruch 10 in der Form eines wässrigen Kosmetikums vom O/W-Typ.

12. Zinkoxidhaltige Verbundpartikel nach einem der Ansprüche 1 bis 7 zur Verwendung in einem therapeutischen Verfahren zur Ultraviolett-Abschirmung.

## Revendications

1. Particules composites contenant de l'oxyde de zinc (1) comprenant chacune :
une matrice dense (10) formée de silice et d'un polysilsesquioxane ; et
des particules d'oxyde de zinc (20) dispersées à l'intérieur de la matrice,
dans lesquelles la teneur en polysilsesquioxane dans la matrice est de 30 % en masse à 70 % en masse.

2. Particules composites contenant de l'oxyde de zinc (1) selon la revendication 1, dans lesquelles
la silice est formée par hydrolyse et condensation avec déshydratation d'un alcoxysilane tétrafonctionnel, et
le polysilsesquioxane est formé par hydrolyse et condensation avec déshydratation d'un alcoxysilane trifonctionnel.

3. Particules composites contenant de l'oxyde de zinc (1) selon la revendication 1 ou 2, dans lesquelles le polysilsesquioxane est un polysilsesquioxane ayant un groupe alkyle lié à un atome de silicium, le groupe alkyle ayant 16 atomes de carbone ou moins.

4. Particules composites contenant de l'oxyde de zinc (1) selon la revendication 1, dans lesquelles le polysilsesquioxane comprend au moins un polysilsesquioxane choisi dans l'ensemble constitué par le polyméthylsilsesquioxane, le polyéthylsilsesquioxane, et le polypropylsilsesquioxane.

5. Particules composites contenant de l'oxyde de zinc (1) selon l'une quelconque des revendications 1 à 4, dans lesquelles la surface spécifique, telle que mesurée par un procédé d'adsorption d'azote, est de 5 m²/g ou moins.

6. Particules composites contenant de l'oxyde de zinc (1) selon l'une quelconque des revendications 1 à 5, lesquelles particules composites contenant de l'oxyde de zinc (1) sont sphériques.

7. Particules composites contenant de l'oxyde de zinc (1) selon l'une quelconque des revendications 1 à 6, dans lesquelles les particules d'oxyde de zinc (20) ont un diamètre moyen de particule de 10 nm à 1000 nm.

8. Composition de protection contre les ultraviolets comprenant les particules composites contenant de l'oxyde de zinc (1) selon l'une quelconque des revendications 1 à 7.

9. Produit cosmétique comprenant les particules composites contenant de l'oxyde de zinc (1) selon l'une quelconque des revendications 1 à 7.

10. Composition de protection contre les ultraviolets selon la revendication 8, ou produit cosmétique selon la revendication 9, comprenant en outre un polymère carboxyvinylique.

11. Composition de protection contre les ultraviolets ou produit cosmétique selon la revendication 10, sous la forme d'un cosmétique aqueux de type huile dans l'eau.

12. Particules composites contenant de l'oxyde de zinc selon l'une quelconque des revendications 1 à 7, pour une utilisation dans un procédé thérapeutique de protection contre les ultraviolets.
